# EUROPEAN PATENT APPLICATION

(11) **EP 4 523 729 A1**
(43) Date of publication of application: **19.03.2025**
(21) Application number: 23803037.3
(22) Date of filing: 12.05.2023
(51) Int. Cl.: A61M 25/14, A61F 2/82

(54) **STENT AND IMPLANTATION DEVICE THEREOF**

(30) Priority: 13.05.2022 CN 202221163845 U
(71) Applicant: Well Lead Medical Co., Ltd., Guangzhou, Guangdong 511400 (CN)
(72) Inventor: WAN, Shaw Pong, Guangzhou, Guangdong 511400 (CN); ZOU, Wenlong, Guangzhou, Guangdong 511400 (CN); DUAN, Songfeng, Guangzhou, Guangdong 511400 (CN)
(74) Representative: Eder Schieschke & Partner mbB
(86) International application number: PCT/CN2023/093871
(87) International publication number: WO 2023/217269

(57) **Abstract**

Provided are a stent and an inserting apparatus thereof. The stent comprises a stent body (1). The stent body (1) includes: a first stent part (2) having at least one lumen (4) for drainage and guidewire passage; and a second stent part (3) having a second cross-section different from a first cross-section of the first stent part (2). The first cross-section of the first stent part (2) transitions to the second cross-section of the second stent part (3) to form the stent body (1), such that the stent body (1) performs drainage or infusion of fluids through the lumen (4). The structure of the stent utilizes a crescent-shape cross-section for the second stent segment (3), employing the siphon principle for drainage. When manufactured from the same material, the crescent-shape or planar second stent segment (3) is thinner, more flexible, and lighter than conventional stents. Additional advantages of the crescent-shape or planar structure include reduced irritation to patient organs and preserved functionality of sphincters.

## Description

### BACKGROUND OF THE APPLICATION

### 1. Technical Field

The present application generally relates to medical devices, and more particularly to a stent and its inserting apparatus.

### 2. Description of Related Art

A double J stent, also known as a pig-tail stent, features curled ends at both extremities. Conventional double J stents provide internal support and drainage functions, and are utilised in various scenarios requiring endoluminal implantation to address conditions like ureteritis. However, in clinical applications, conventional double J stents can cause significant patient discomfort due to the expansion of the stent's tubular portion, resulting in compression of surrounding tissues. Particularly, existing ureteral or biliary stents may induce considerable discomfort when implanted, including pains, frequent micturition, and urinary urgency due to bladder irritation. Besides, known stents usually have two ends each including a coiled segment. When such a stent is placed in a patient, its length within the renal pelvis is fixed, while its remaining part resides in the bladder. This might not cause irritation to patients with long ureters; however, for patients with short ureters, the thick and improperly coiled end can cause substantial discomfort.

China Patent No. CN112107781A discloses a ureter stent comprising a first body communicating with a second body arranged in the ureter, and a bladder section communicating with the second body and arranged in the bladder. The first body is an elastic tube horizontally curled in an Archimedes spiral shape. The first body of the ureteral stent, arranged in the renal pelvis, allows for adjustment of the overall stent length when placed in the human body by modifying the number of curled circles. This design enables a single stent length to accommodate patients with different ureteral lengths. The tape measure-like structure of the first body, together with the in-bladder section, significantly reduces the stent's size within the bladder, thereby minimising bladder irritation caused by the stent. However, this prior-art device has the disadvantage of an integrated transition that can still irritate the bladder upon placement, particularly as the curled tubing occupies considerable space, compressing the patient's sphincter and consequently impeding both fluid drainage and infusion.

China Patent No. CN111494778B discloses a ureteral stent capable of visualisation. The ureteral stent is made of a material comprising a first radiocontrast agent and a second radiocontrast agent, respectively distributed in different areas of the ureteral stent. This embodiment facilitates clear visualisation of the ureteral stent's position during X-ray or ultrasound examinations, enhancing medical observation. Meanwhile, it allows flexible selection of examination modalities based on individual patient requirements, eliminating the need for stent replacement during procedures and thereby reducing patient discomfort and burden. However, while this known device employs radiocontrast agents to observe the stent's location within a patient, it does not address the issue of bladder irritation. Besides, it is designed exclusively for drainage and is ineffective for fluid infusion. This necessitates replacing the known stent with a different ureter stent whenever fluid infusion is required and adds discomfort and burden to the patient.

Since there may be discrepancy between the prior art comprehended by the applicant and that known to the patent examiners, and since numerous details and disclosures from literatures and patent documents have been referred to but not exhaustively recited here, it should be noted that the present application encompasses the technical features of all these prior-art works, and the applicant reserves the right to supplement the application with additional related prior art and technical features according to relevant regulations.

### SUMMARY OF THE APPLICATION

In view of the shortcomings of the prior art, the present application provides a stent comprising a stent body that includes:
- a first stent segment having at least one lumen for drainage and/or guidewire passage; and
- a second stent segment having a second cross-section that differs from a first cross-section of the first stent segment,
wherein the first cross-section of the first stent segment transitions to the second cross-section of the second stent segment to form the stent body, allowing the stent body to perform drainage and/or infusion of fluids through the lumen. The stent of the present application is structurally characterized by the crescent-shape cross-section of the second stent segment, which facilitates siphon drainage. Compared to conventional stents made of the same material, the crescent-shape or planar second stent segment disclosed herein is thinner, more flexible, and lighter. The crescent-shape or planar structure also advantageously reduces irritation to the patient's organs and preserves functions of the patient's sphincter.

According to a preferred mode, a connecting portion between the first cross-section and the second cross-section reduces irritation to a patient undergoing drainage by decreasing the compression force exerted by the patient's sphincter on the second stent segment, thereby preserving sphincter function.

According to a preferred mode, the first stent segment has a free end coiled in a first direction to form a deformable first loop, and the second stent segment has a free end coiled in a second direction to form a deformable second loop. Preferably, a first radial surface of the first loop and a second radial surface of the second loop are separated by a first angle ranging from 0° to 180°. Provision of the first loop and the second loop allows for better fixation of the stent in patients with varying body lumen lengths.

According to a preferred mode, the second cross-section comprises a drainage structure with a crescent-shape cross-section or a planar form. Preferably, the ratio of the area of the second cross-section to the area of the first cross-section defines completeness of the stent body. Based on the selected completeness, the drainage efficacy of the stent body is inversely proportional to patient comfort.

According to a preferred mode, the connecting portion comprises a first connecting end of the first stent segment and a second connecting end of the second stent segment, wherein the first connecting end of the first stent segment forms an oblique end that defines a tangent plane intersecting the axis of the first stent segment at a second angle. Preferably, the second angle ranges from 0° to 90°, ensuring that the connecting portion between the first and second crosssections has a smooth profile. The inclined transition eliminates edges and corners, thereby ensuring patient comfort. A 90° provides optimal connection between the first and second stent segments, maximizing the pushing action of a pusher. Preferably, the inclination angle may be 30°, 45°, or 60°. At 60°, the pusher achieves satisfactory pushing efficiency while maintaining acceptable patient comfort. At 45°, the pusher balances competent pushing efficiency and sufficient patient comfort. At 30°, the pusher provides satisfactory patient comfort with acceptable pushing efficiency.

In view that conventional ureteral or biliary stents, when placed, can cause apparent discomfort including pains, frequent micturition, and urinary urgency due to bladder irritation, the present application provides a stent for which existing positioners designed for conventional ureteral or biliary stents are not applicable because of the specially designed cross-section of the lower end of the disclosed stent.

The present application further provides an inserting apparatus for placing the stent, comprising at least:
- an inner tube, for drainage and/or passage of a guidewire; and
- an outer tube, for separating the stent body from the guidewire.
Preferably, in use, after the inner tube is placed within the outer tube and inserted into a lumen formed in the first stent segment of the stent body, a fastener secures the drainage structure in the second cross-section of the second stent segment of the stent body to the outer tube. The inserting apparatus of the present application, when assembled with the stent, not only serves as a catheter but also facilitates injection of radiocontrast agents and infusion of fluids.

According to a preferred mode, the inner tube comprises:
- an inner tube body serving as a lumen for drainage and guidewire passage;
- a first Luer connector acting as a port of the inner tube for connection to the outer tube; and
- a second Luer connector for detachable connection to an external connector.
Preferably, the end of the inner tube distant from the first and second Luer connectors is inserted into the lumen in the first stent segment of the stent body for drainage and/or guidewire passage. The assembled inserting apparatus and the stent of the present application allow for simple and efficient placement of the stent into a patient and may optionally be used as a ureteral or biliary catheter for injection of radiocontrast agents or other fluids.

According to a preferred mode, the inner tube further comprises:
- a first radiopaque marker located at one end of the inner tube body distant from the first and second Luer connectors; and
- a second radiopaque marker located between the first radiopaque marker and the firstLuer connector.
Preferably, the first and second radiopaque markers are used for positioning the stent body during a stenting procedure.

According to a preferred mode, the outer tube comprises:
- an outer tube body for accommodating the inner tube body;
- a handle located at an end of the outer tube body and configured to advance or retract the outer tube; and
- a third Luer connector for connecting to the inner tube.
Preferably, the third Luer connector and the first Luer connector are connected so that when the inner tube in inserted to and assembled with the outer tube, the inner tube and the outer tube are positionally fixed relative to each other.

According to a preferred mode, the surfaces of the inner tube and the outer tube are provided with a coating that improves comfort and safety of the patient receiving the stent using the inserting apparatus.

### BRIEF DESCRIPTION OF THE DRAWINGS

FIG. 1 is a schematic view of a stent according to an embodiment of the present application;
FIG. 2 is a schematic view of a stent according an embodiment of the present application, wherein the stent has a first angle of 90° and single-turn loops;
FIG. 3 is a schematic view of a stent according to an embodiment of the present application, wherein the stent has a first angle of 90° and double-turn loops;
FIG. 4 is a schematic view of a stent according to an embodiment of the present application, wherein the stent has a first angle of 90° and single-turn loops while being equipped with a retention assembly;
FIG. 5 is a schematic view of a stent according to an embodiment of the present application, wherein the stent has a first angle of 180° and single-turn loops;
FIG. 6 is a schematic view of a stent according to an embodiment of the present application, wherein the stent has a first angle of 180°and double-turn loops;
FIG. 7 is a schematic view of a stent according to an embodiment of the present application, wherein the stent has a first angle of 180° and single-turn loops while being equipped with a retention assembly;
FIG. 8 is an applied view of a stent according to an embodiment of the present application;
FIG. 9 is a zoom-in view of a connecting portion in a stent according to an embodiment of the present application;
FIG. 10 is a schematic view of a stent according to an embodiment of the present application, wherein the stent has a planar drainage structure;
FIG. 11 is a schematic view of an inner tube of a stent according to an embodiment of the present application;
FIG. 12 is a cross-sectional view of an outer tube of a stent according to an embodiment of the present application;
FIG. 13 is a schematic view of a stent according to an embodiment of the present application, wherein the second stent segment and the outer tube are assembled;
FIG. 14 is a perspective view according to an embodiment of the present application showing the stent and the inserting apparatus are assembled;
FIG. 15 is a side view according to an embodiment of the present application showing the stent and the inserting apparatus are assembled;
FIG. 16 is a front view according to an embodiment of the present application showing the stent and the inserting apparatus are assembled; and
FIG. 17 is a cross-sectional view according to an embodiment of the present application showing the stent and the inserting apparatus are assembled.

### List of Reference Signs

| | | | |
|---|---|---|---|
| 1: | stent body | 11: | first radiopaque marker |
| 2: | first stent segment | 12: | second radiopaque marker |
| 3: | second stent segment | 13: | inner tube body |
| 4: | lumen | 14: | first Luer connector |
| 5: | first loop | 15: | second Luer connector |
| 6: | second loop | 16: | third Luer connector |
| 7: | retention assembly | 17: | outer tube body |
| 8: | connecting portion | 18: | handle |
| 9: | inner tube | 19: | fastener |
| 10: | outer tube | | |

### DETAILED DESCRIPTION OF THE APPLICATION

The present application will be described in detail with reference to the accompanying drawings.

### Embodiment 1

The present application relates to a stent, which at least comprises a stent body 1 having a first stent segment 2 that transitions to a second stent segment 3. The first stent segment 2 has at least one lumen 4 for drainage or guidewire passage. A connecting portion 8 between the first stent segment 2 and the second stent segment 3 is arranged such that it transitions from a first cross-section of the first stent segment 2 to a second cross-section of the second stent segment 3. The second stent segment 3 defines therein at least one recess communicating with and adapted to the lumen 4. Preferably, the first stent segment 2 is tubular and defines therein the lumen 4, so as to form a continuous tube. The first stent segment 2 is to be inserted into a body lumen of a patient to act as a ureteral or biliary catheter. The second stent segment 3 extends from the first stent segment 2 but has its cross-section changed. The first stent segment 2 has a cross-section that is preferably circular in shaped. With the presence of the lumen 4, the cross-section may be regarded as ring-shaped. However, for clarity, the cross-section is herein referred to as a circular cross-section. The second stent segment 3 has a non-circular cross-section, which is a result of transition from the circular cross-section of the first stent segment 2. Preferably, the cross-section of the second stent segment 3 is preferably crescent-shape. The crescent shape and the circular shape may be of any suitable proportion as determined by anatomical features of the patient. For example, for average patients, the crescent shape may be a 50% crescent shape, or a semicircle, or a half ring. If the body lumen of the patient is relatively narrow, the crescent shape is preferably one greater than 50% so as to ensure entrance of the stent body 1. If the body lumen of the patient is relatively loose, the crescent shape is preferably one smaller than 50% so as to ensure comfort of the patient. It is to be noted that first cross-section may be a circular, elliptical, or oval, tubular cross-section. For conciseness, other possible shapes for the cross-section that are not recited herein shall all fall within the scope of the present application. The second cross-section is an incomplete form of the first cross-section. Its integral form may have a circular, elliptical, or oval, tubular cross-section, as described previously. Specifically, the first cross-section is elliptical and the second cross-section are each in the shape of a partial ellipse, whose completeness may be of 50%, smaller than 50%, or greater than 50%. An elliptical cross-section is favorable to comfort of patients because it brings less compression to the internals of the patient, yet it is unfavorable smooth drainage. Therefore, selection of the shape shall be made according to anatomical features of patients. For example, for a patient with a relative narrow ureter, a stent having a cross-section with fewer completeness is preferable. In another example, the first cross-section is oval, and the second cross-section is in the shape of a partial ovality, whose completeness may be of 50%, smaller than 50%, or greater than 50%. An oval structure is narrow at the top and wide at the bottom and has a smooth profile, so it contributes to both smooth drainage and satisfying patient comfort. Particularly, the curved counter of an ovality ensures smooth flow of fluid during drainage without obstruction. It is understandable that, on the basis of the foregoing second cross-section, the second cross-section has a lateral edge formed with a radius (R) to prevent damage to the patient's body lumen. Specifically, the R angle is formed at the edge of the second cross-section where the completeness is broken.

According to a preferred mode, the cross-section of the second stent segment 3 may alternatively be rectangular. In this case, it serves as a planar drainage piece of the stent for fluid drainage. The lumen 4 is for guidewire passage. The second stent segment 3 has its end distant from the lumen 4 being straight, J-shaped, or pig-tail-shaped, so as to facilitate fixation of the stent and prevent the stent from displacement. The first stent segment 2 allows fluid to flow out from the lumen 4 at the center or from the periphery of the stent body 1. The second stent segment 3 has a different shape of the cross-section. Particularly, the second stent segment 3 may be a cross-sectionally crescent-shape or planar drainage piece for siphon-based drainage. Advantageous of such a design include brining less irritation to patient internals and maintaining the sphincter functional. In the embodiment, the second stent segment 3 of the disclosed stent has a crescent-shape cross-section for siphon-based drainage. The crescent-shape or planar second stent segment 3 of the present application is tinner, softer, and lighter as comparted to a conventional counterpart made of the same material. The crescent-shape or planar structure also advantageously reduces irritation to organs of its patient and preserves functions of the sphincter of the patient.

According to a preferred mode, the connection between the first stent segment 2 and the second stent segment 3 may be achieved through split or integrated means, or any other suitable means. As the first stent segment 2 enters the body lumen of the patient, the second stent segment 3 is pulled into the body lumen of the patient. The split connection may be implemented using detachable medical parts, such as clips, threads, etc. The integrated connection may be achieved through thermal welding, injection molding, or ultrasonic welding.

According to a preferred mode, the free end of the first stent segment 2 coils up in a first direction to form a deformable first loop 5, and the free end of the second stent segment 3 coils up in a second direction to form a deformable second loop 6. The first radial surface of the first loop 5 and the second radial surface of the second loop 6 jointly include the first angle ranging between 0 and 180 degrees. In the present application, the first direction refers to a direction extending from the first stent segment 2 to the axis of the second stent segment 3. The second direction refers to a direction opposite to the first direction. The first radial surface and the second radial surface include planes extending beyond the first loop 5 and the second loop 6. The angle defined by the first radial surface and the second radial surface is the first angle. Preferably, the first radial surface and the second radial surface extend through the straight line in which the stent body 1 is located. In other words, the first radial surface and the second radial surface rotate about the same axis, thereby forming the first angle that can vary in a certain plane. Preferably, the first loop 5 and the second loop 6 each have a diameter ranging between 10 mm and 30 mm. The first loop 5 and the second loop 6 are for providing additional stretching length that facilitates placement of the stent into the body of a patient. Preferably, the first loop 5 and the second loop 6 further facilitate fixation in the bladder and the renal calyces of a patient. Preferably, first stent segment 2 has a diameter ranging between 1.0 mm and 3.0 mm.

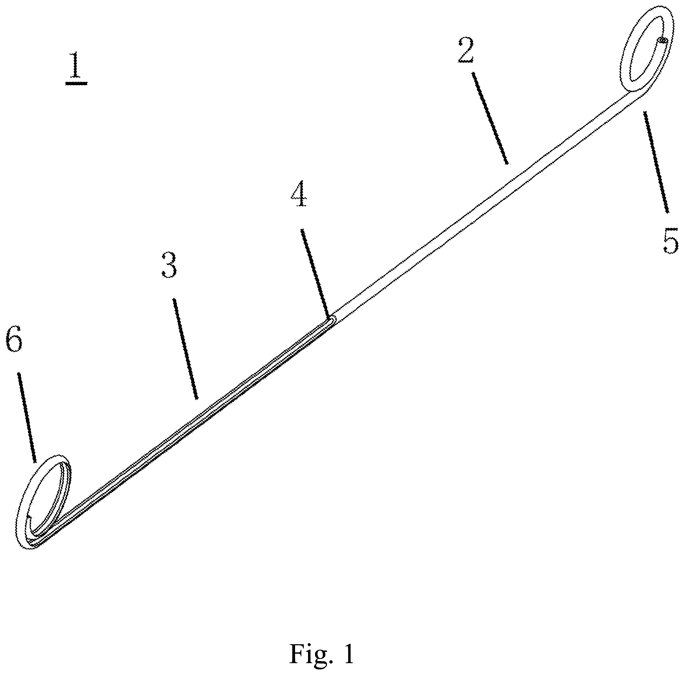

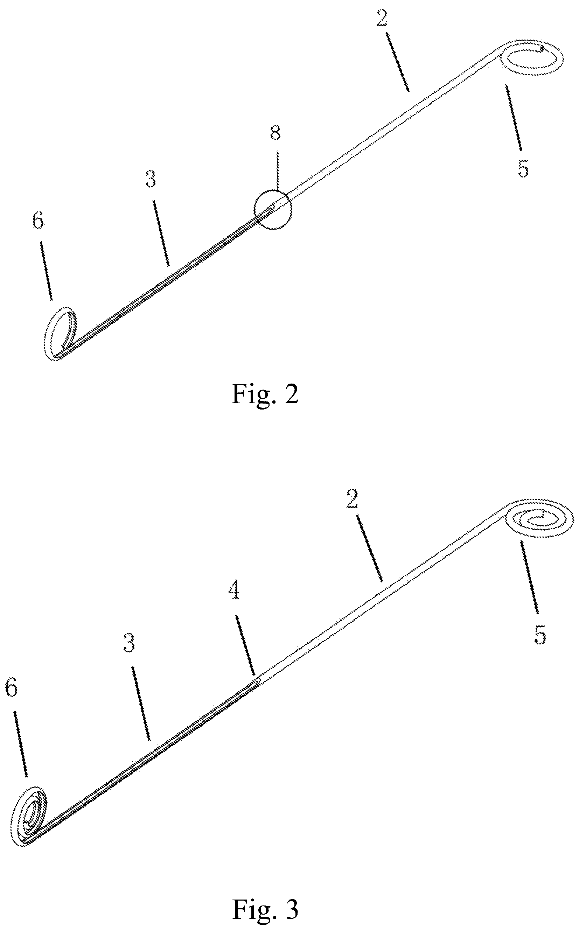

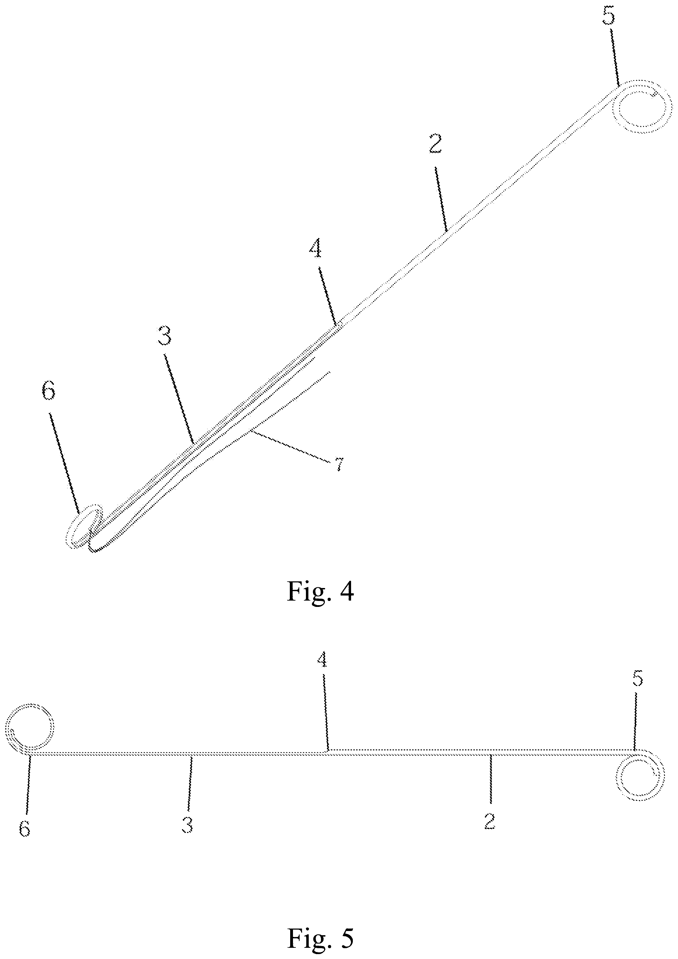

The first angle is the angle between the first loop 5 on the first stent segment 2 and the second loop 6 on the second stent segment 3 when they are projected into the same plane. Specifically, the plane is a cross-section of the first stent segment 2 or the second stent segment 3. For example, as shown in FIG. 5, the cross-section of the first stent segment 2 or the second stent segment 3 is a plane that faces inward and is perpendicular to the first stent segment 2 and the second stent segment 3. The first loop 5 and the second loop 6 have opposite projection directions, forming a first angle of 180°. As shown in FIG. 1, the angle between the first loop 5 and the second loop 6 of the stent body 1 is of zero. In other words, the first angle is zero. As shown in FIG. 2, the first loop 5 and the second loop 6 of the stent body 1 are perpendicular to each other or different from teach other by 90°, so the first angle is of 90°. As shown in FIG. 5, the first loop 5 and the second loop 6 of the stent are parallel to each other or different from teach other by 180°, so the first angle is of 180°. It is appreciable that the first angle may be of any other value not enumerated herein. Provision of the loops at the two ends eliminates difficulty in fixing the stent in patients having different body lumen lengths. The loops at the two ends facilitate fixation of the stent body 1. Preferably, the first loop 5 and the second loop 6 generally each have a single turn or two turns, while any greater number of turns may be possible with the first loop 5 and the second loop 6. In the present application, by selecting the first angle for the first loop 5 and the second loop 6, respectively, the stent can be made to fit patients with different physiological features and be secured at the set location without the risk of displacement.

According to a preferred mode, the first radial surface of the first loop 5 and the second radial surface of the second loop 6 are overlapped in one plane. Alternatively, the first radial surface of the first loop 5 and the second radial surface of the second loop 6 are perpendicular to each other. Thereby, a stent whose first angle between the first loop 5 and the second loop 6 is 0°, 90°, or 180° can be formed.

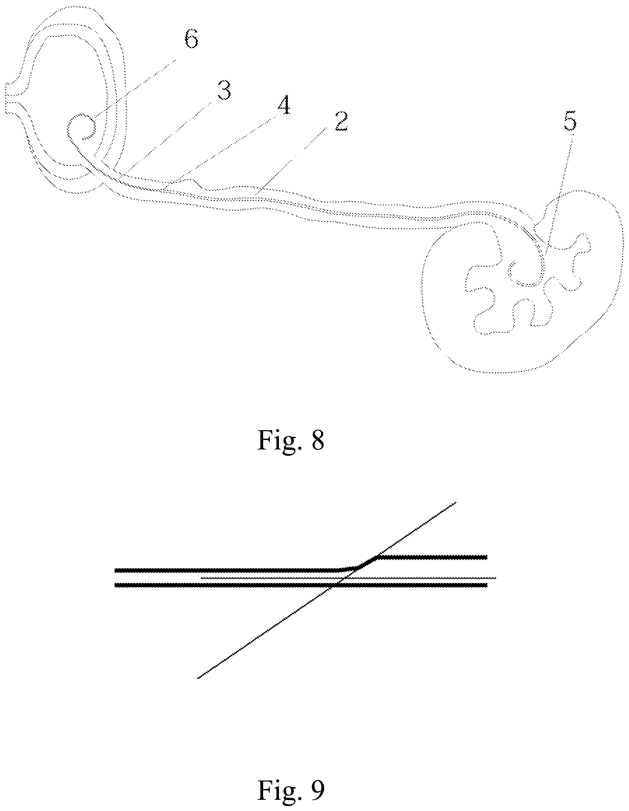

According to a preferred mode, the connecting portion 8 includes the first connecting end of the first stent segment 2 and the second connecting end of the second stent segment 3. The first connecting end of the first stent segment 2 has an oblique end. The tangent plane of the oblique end intersects the axis of the first stent segment 2 and the second angle is formed therebetween. The second connecting end and the first connecting end of the second stent segment 3 are such connected with a smooth transition. As shown in FIG. 9, the angle included by the two straight lines is the second angle. The second angle may be 90°or preferably an angle of inclination. The inclination at the transition eliminates any corner at the site, thereby improving patient comfort. On the other hand, the 90° angle is favorable to the connection between the first stent segment 2 and the second stent segment 3, thereby maximizing pushing efficiency of the pusher. Preferably, the inclination is of 30°or 45°or 60°. When the inclination angle is 60°, the pusher can have satisfying pushing efficiency and acceptable patient comfort. When the inclination angle is 45°, the pusher supports balance between competent pushing efficiency and sufficient patient comfort. When the inclination angle is 30°, the pusher can have satisfying patient comfort and acceptable pushing efficiency.

According to a preferred mode, the first loop 5 of the first stent segment 2 has its end formed with an opening that is communicating with the lumen. Alternatively, the end of the first loop 5 is formed as a closed end.

According to a preferred mode, the first stent segment 2 is provided with a solid wall or pores for discharging water, and the second stent segment 3 is provided with a solid wall, pores and/or a longitudinal ridge for discharging water. Preferably, the diameter of the first stent segment 2 may be adaptively selected so as to better match the body lumen, such as the ureter or the bile duct. The diameter of the second stent segment 3 may be selected according to the selection of the diameter of the first stent segment 2, as to better match the body lumen. The materials used to make the first stent segment 2 and the second stent segment 3 may be flexible materials or semi-rigid materials or any combination thereof. The material may be dissolvable or undissolvable. Usable materials include and are not limited to PVC, latex, silicone, PU, TPE, and organic dissolvable materials, such as polyglycolic acido, porcine small intestinal submucosa, and exogenous collagen. The first stent segment 2 and the second stent segment 3 may be made of the same material or different materials.

According to a preferred mode, the end of the second stent segment 3 distant from the first stent segment 2 is provided with a retention assembly 7 that is configured to be left outside the body lumen of the patient. The retention assembly 7 is used to remove the stent body 1 from the body lumen of the patient.

According to a preferred mode, the stent body 1 has gradient marks arranged in its length direction.

According to a preferred mode, the stent further comprises a separate pusher with a tubular structure like the first stent segment 2. The pusher is used to place the stent body 1 into the body lumen of the patient. The pusher has at least one second lumen 4 for the guidewire or another relevant device to pass through. The pusher and the stent body 1 are detachably connected to each other. The stent body 1 and the pusher are formed as separate components that have detachable connecting parts so as to facilitate insertion of the stent body 1 into the body lumen. The detachable connecting parts may be connected in a mechanical or magnetic way. The pusher may be made of a hard, semi-rigid, or flexible material. To a user of the device, the distal end of the pusher shall be in contact with the proximal end of the stent body 1. The distal end of the pusher may be horizontal or inclined so ta to better connect the proximal end of the stent body 1 with respect to a user of the device.

According to a preferred mode, each of the first stent segment 2 and the second stent segment 3 is covered by a layer of hydrophilic or antimicrobial or anti-calcification coating. The pusher is covered by a layer of hydrophilic or antimicrobial coating. The hydrophilic coating serves to decrease the coefficient of friction between the stent body 1 and the contacting part of the patient. This reduces discomfort caused when the stent enters the patient's body and prevent undue friction from injuring the internals or blood vessels of the patient. Meanwhile, the applied antimicrobial or anti-calcification coating also helps to prevent infection and thereby rendering the tenting operation safer to the patient.

The working principles and usage of the stent of the present application will be further explained for clarity.

The first stent segment 2 of the stent body 1 is generally pushed forward using a guidewire. In the process that the guidewire is inserted into the body lumen of the patient, it is important to keep the first loop 5 of the first stent segment 2 upright so that the second loop 6 of the second stent segment 3 can simply follow the first stent segment 2 to enter the body lumen of the patient. Once the stent body 1 enters the body lumen of the patient, the first loop 5 can unfold automatically. When the first stent segment 2 is positioned as planned for treatment, a pusher will usually be used to stable the stent body 1 and then the guidewire can be removed. The auxiliary lumen provided in the pusher (i.e. the lumen 4) is for injection of a radiocontrast agent or insertion of other relevant devices. Connection between the distal end of the pusher and the proximal end of the stent body 1 can be used as the pushing point of the stent body 1 and the pusher.

### Embodiment 2

The present embodiment provides supplements to the foregoing embodiment and drawings (specifically, various models of the disclosed stent). Repetitive descriptions will be omitted herein for conciseness.

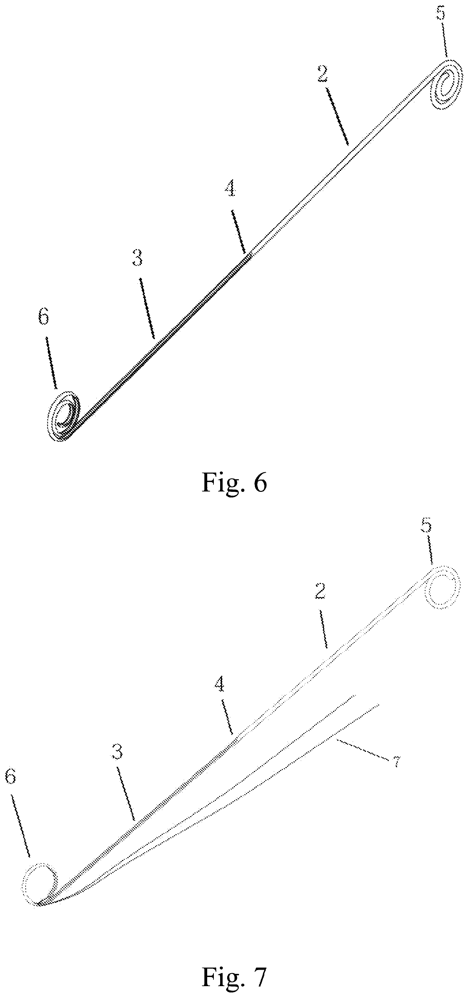

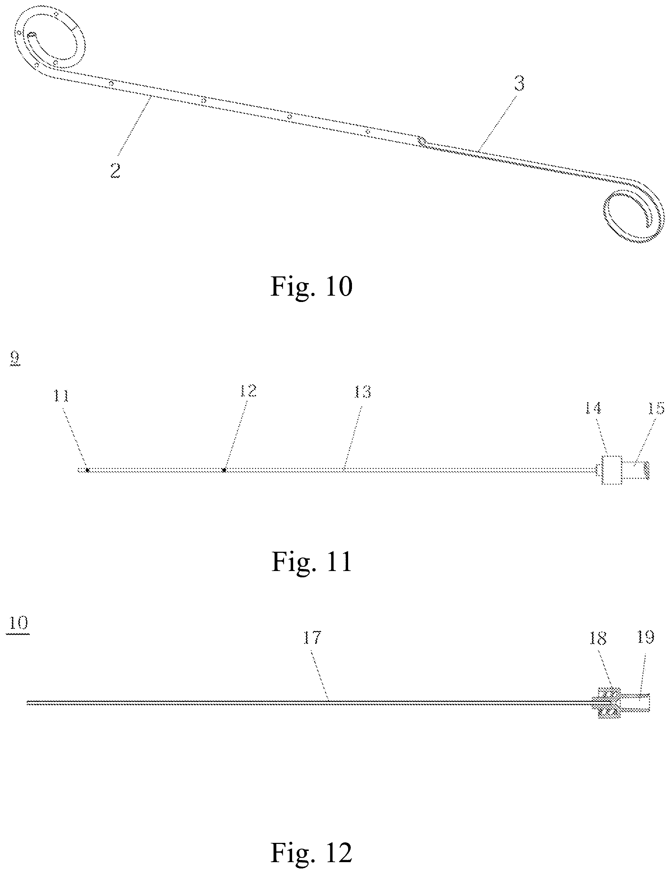

According to a preferred mode, FIG. 1 depicts a preferred embodiment of the stent of the present application. As shown, the stent is configured with a first angle of 0° and single-turn loops. This represents the basic design of the present application. FIG. 2, 3, and 4 each depict a stent with a first angle of 90°, differing in the number of loops and the presence or absence of a retention assembly. FIG. 5, 6, and 7 each show a stent with the first angle of 180°, similarly differing in the number of loops and the presence or absence of a retention assembly. While these examples represent possible alternative structures of the present application that may be particularly suitable for practical use, other alternative structures may exist, such as those with different configurations of the first angle, second angle, and number of loop turns. FIG. 8 illustrates an applied view of the present application in practical use. Preferably, the stent is positioned in the bladder and renal calyces of a patient. FIG. 9 provides a zoom-in view of the connecting portion of the present application. The angle included by the two straight lines is the second angle. FIG. 10 shows another preferred embodiment of the stent of the present application. In additional to the single-turn structure of each of the first loop 5 and the second loop 6, the present application further proposes a multi-turn structure for shaping the first loop 5 and the second loop 6. The required length of a stent varies with anatomical features of patients who use the stent. The first loop 5 and the second loop 6 with a multi-turn structure can provide additional elongation capacity and make the stent adaptable to various patients with different anatomical features. Particularly, depending on the body height or the age of the patient, an excessive long stent can cause discomfort while an excessive short stent may fail to properly anchor itself in the bladder and renal calyces of the patient. The additional turns provided by the multi-turn structure allow the stent to better anchor itself in the bladder and renal calyces of a patient and endow the stent with additional elongation capacity.

### Embodiment 3

The present embodiment provides further improvements on the previous embodiments. Repetitive details are omitted from the description thereof.

The present embodiment relates to an inserting apparatus for placing the stent as described previously. Existing ureteral stents or biliary stent, when placed in the body, may cause significant discomfort, such as pains, frequent micturition, and urinary urgency due to bladder irritation. The embodiments of the present application as described previously provide a comfortenhanced stent, but the stent's unique cross-sectional design at its lower end renders it incompatible with existing introducers for conventional ureteral or biliary stents. Therefore, the present application further provides an inserting apparatus configured to place the disclosed stent into the body of a patient. Additionally, conventional ureteral and biliary stents are only intended for post-operative support and drainage. When combined with the stent, the inserting apparatus of the present embodiment serves as a catheter that facilitate the injection of radiocontrast agent and infusion of fluids.

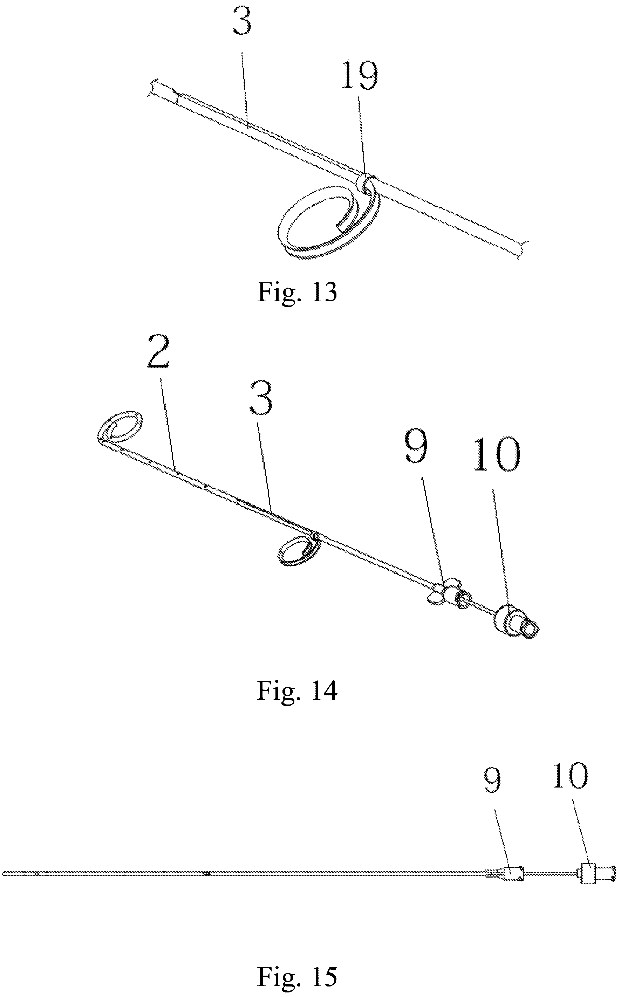

According to a preferred mode, as shown in FIG. 15, the inserting apparatus comprises at least an inner tube 9 and an outer tube 10. FIG. 15 shows the inner tube 9 and the outer tube 10 in an assembled state. Preferably, the inner tube 9 and the outer tube 10 are coaxially aligned and can be connected via Luer connectors. Preferably, the concentric lumen of the inner tube 9 is configured for guidewire passage or fluid injection. The outer tube 10 serves as a pusher tube to facilitate the separation of the stent from the guidewire. As shown in FIG. 11, preferably, the inner tube 9 includes a first radiopaque marker 11, a second radiopaque marker 12, an inner tube body 13, a first Luer connector 14, and a second Luer connector 15. Preferably, the end of the inner tube 9 distant from the first Luer connector 14 and the second Luer connector 15 is tapered for easy insertion into the lumen 4 of the stent. The stent and the inner tube 9 are thereby combined as an integral unit. At this point, the second Luer connector of the inner tube 9 can be used to inject radiocontrast agent or act as a ureteral or biliary catheter for input of other fluids. When the inner tube 9 and the outer tube 10 are connected together through the Luer connectors, pushing the handle 18 on the outer tube 10 firmly can separate the stent from the inner tube 9, leaving the stent in the patient's body. Preferably, the first radiopaque marker 11 is located at the end of the inner tube body 13 distant from the first Luer connector 14 and the second Luer connector 15. The first radiopaque marker 11 and the second radiopaque marker 12 are for indicating the position of the stent during the stenting procedure. In the present technical scheme, the first radiopaque marker 11 and the second radiopaque marker 12 on the inner tube body 13 may each be an inlaid metal piece in the form of a ring, wire, or spring, as long as they are conspicuous in X-ray images. By inlaying the first radiopaque marker 11 and the second radiopaque marker 12 at different locations on the inner tube body 13, medical staff can accurately locate the inner tube 9 radiographically. With the first radiopaque marker 11 and the second radiopaque marker 12, precise insertion can be achieved radiographically. The present application thereby improves clinical catheter insertion in terms of positioning accuracy and success rate. Preferably, the first radiopaque marker 11 and the second radiopaque marker 12 are inlaid on the inner tube body 13 as metal rings, metal wires, or metal springs. More preferably, the first radiopaque marker 11 and the second radiopaque marker 12 may be made of one of a nickel-titanium alloy, tungsten, or platinum. Preferably, the first Luer connector 14 and the second Luer connector 15 are both located at the end of the inner tube 9 distant from the first radiopaque marker 11. The first Luer connector 14 is positioned closer to the inner tube body 13 compared to the second Luer connector 15. Preferably, the inner tube 9 and the outer tube 10 coated to enhance patient comfort and safety. The coating may be an ultra-lubricating coating, and its material comprises at least one of polyvinylpyrrolidone (PVP), polyacrylamide (PAM), hydrophilic silver nitrate, or other hydrophilic ultra-lubricating materials. Preferably, the outer surface of the outer tube 10 is also provided with distance markers to facilitate proper insertion by medical staff. As shown in FIG. 12, preferably, the outer tube 10 comprises an outer tube body 17, the handle 18 and the third Luer connector 16. Preferably, the handle 18 is flat for easy gripping. Preferably, the outer tube 10 further has an end for the fastener 19 to pass through to secure the stent to the outer tube 10, thereby preventing displacement of the stent's end. Preferably, the fastener 19 may comprise a radiopaque material to facilitate positioning by medical staff. FIG. 13 schematically displays the fastener 19 securing the second stent segment 3. Preferably, the fastener 19 is formed as a sleeve or a clamp for fixation. Preferably, the first Luer connector 14 and the second Luer connector 15 are the male and female parts of the Luer connection, respectively. The first Luer connector 14 serves as the front port of the inner tube 9 to connect with the third Luer connector 16 of the outer tube 10. Thereby, when the inner tube 9 is inserted into the outer tube 10, the inner tube 9 and the outer tube 10 are fixed. The second Luer connector 15 may be detachably connected to a standard connector for convenient fluid infusion. In addition, after the combination is formed, a matching guidewire and radiocontrast agent can be selected. The combination of the inserting apparatus and the stent of the present application enables easy and efficient placement into the patient's body, and serves as a catheter for the ureter or bile duct, allowing for convenient injection of radiocontrast agents and other fluids.

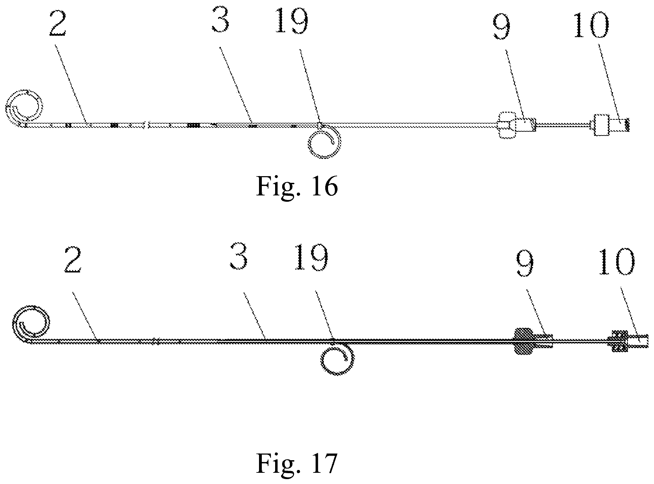

The working principles and use of the stent of the present application will be further explained for clarity.
1. After the safety guidewire is left in the body of the patient, the stent and the inserting apparatus are assembled together. FIG. 14 shows the assembled state of the stent, inner tube 9, and outer tube 10. FIG. 16 and FIG. 17 are front and cross-sectional views of the combination of the stent and the inserting apparatus, respectively. Preferably, the inner tube 9 is inserted into the outer tube 10, and then they are fixed through the first Luer connector 14 and the third Luer connector 16. Afterward, the taped part at the front end of the inner tube 9 is inserted into the lumen 4 in the first stent segment 2 of the stent. The crescent-shape drainage structure or planar drainage structure or otherwise shaped drainage structure of the stent is fixed to the outer tube 10 by the fastener 19.
2. The guidewire is inserted from the front end of the inserting apparatus. The inserting apparatus is advanced along the guidewire. In this process, the locations of the first radiopaque marker 11 and the second radiopaque marker 12 are checked radiographically, and distance markers on the outer tube 10 are observed to confirm whether the stent has reached the intended location. If radiocontrast agent is required for radiographic confirmation, the radiocontrast agent maybe injected through the second Luer connector 15 at the back end of the inner tube 9. The radiocontrast agent then flows along the inserting apparatus and the stent to flow out the lumen 4 to make the corresponding tissues visible radiographically.
3. With the stent positioned at the intended location, leaving the stent in the ureter of the patient can be easily achieved by confirming that the inner tube 9 and the outer tube 10 are assembled together and pushing the handle 18 away from the stent. The second stent segment 3 of the stent will separate from the inner tube 9 automatically. It is preferable that the pulled inner and outer tubes 9, 10 are checked for completeness, and proper placement of the stent is confirmed again radiographically.

Throughout the present application, features introduced by the term "preferably" are optional but not mandatory, and the applicant also reserves the right to withdraw or omit such preferred features at any time.

It is to be noted that the particular embodiments described previously are exemplary. People skilled in the art, with inspiration from the present application, would be able to devise various solutions, all of which fall within the scope of the present application and are protected by it. Further, people skilled in the art would appreciate that the description and accompanying drawings provided herein are illustrative and form no limitation to any of the appended claims. The scope of the present application is defined by the appended claims and equivalents thereof. The present application provided herein encompasses multiple inventive concepts, indicated by phrases such as "preferably", "according to a preferred mode" and "optionally", each denoting a distinct concept disclosed in the respective paragraph. The applicant reserves the right to file one or more divisional applications based on each inventive concept.

## Claims

1. A stent, comprising a stent body (1) that includes:
a first stent segment (2) having at least one lumen (4) for drainage and/or guidewire passage; and
a second stent segment (3) having a second cross-section that differs from a first cross-section of the first stent segment (2),
wherein the first cross-section of the first stent segment (2) transitions to the second cross-section of the second stent segment (3) to form the stent body (1), so that the stent body (1) performs drainage or infusion of fluids through the lumen (4).

2. The stent of claim 1, wherein during drainage, a connecting portion (8) connecting the first cross-section and the second cross-section reduces irritation to a patient by decreasing a compression force exerted by a sphincter of the patient on the second stent segment (3).

3. The stent of claim 1 or 2, wherein a free end of the first stent segment (2) is curved in a first direction to form a deformable first loop (5), and a free end of the second stent segment (3) is curved in a second direction to form a deformable second loop (6), wherein a first radial surface of the first loop (5) and a second radial surface of the second loop (6) are separated from each other by a first angle ranging from 0° to 180°.

4. The stent of any of claims 1 to 3, wherein the second cross-section comprises a drainage structure having a crescent-shape cross-section or a planar shape,
wherein an area ratio between the second cross-section and the first cross-section defines a completeness of the stent body (1), and based on the selection of completeness, the drainage efficiency through the stent body (1) is inversely proportional to patient comfort.

5. The stent of any of claims 1 to 4, wherein the connecting portion (8) comprises a first connecting end of the first stent segment (2) and a second connecting end of the second stent segment (3), wherein the first connecting end of the first stent segment (2) is formed as an oblique end that defines a tangent plane intersecting an axis of the first stent segment (2) to form a second angle,
wherein the second angle ranges from 0° to 90° so that the connecting portion (8) connecting the first cross-section and the second cross-section has a smooth profile.

6. An inserting apparatus for placing a stent, the inserting apparatus comprising at least:
an inner tube (9) for drainage and guidewire passage; and
an outer tube (10) for separating a stent body (1) of the stent from the guidewire,
wherein in use, after the inner tube (9) is placed within the outer tube (10) and inserted into a lumen (4) formed in a first stent segment (2) of the stent body (1), a fastener (19) secures a drainage structure in a second cross-section of a second stent segment (3) of the stent body (1) to the outer tube (10).

7. The inserting apparatus of claim 6, wherein the inner tube (9) comprises:
an inner tube body (13) serving as a lumen for drainage and guidewire passage;
a first Luer connector (14) serving as a port of the inner tube (9) for connection to the outer tube (10); and
a second Luer connector (15) for detachable connection to an external connector,
wherein one end of the inner tube (9) distant from the first Luer connector (14) and the second Luer connector (15) is inserted into the lumen (4) in the first stent segment (2) of the stent body (1) for drainage or guidewire passage.

8. The inserting apparatus of claim 6 or 7, wherein the inner tube (9) further comprises:
a first radiopaque marker (11) located at one end of the inner tube body (13) distant from the first Luer connector (14) and the second Luer connector (15); and
a second radiopaque marker (12) located between the first radiopaque marker (11) and the first Luer connector (14),
wherein the first radiopaque marker (11) and the second radiopaque marker (12) are used for positioning the stent body (1) during a stenting procedure.

9. The inserting apparatus of any of claims 6 to 8, wherein the outer tube (10) comprises:
an outer tube body (17) for accommodating the inner tube body (13); and
a handle (18) located at an end of the outer tube body (17) and configured to be operated to advance or retract the outer tube (10), and
a third Luer connector (16), for connecting the inner tube (9),
wherein the third Luer connector (16) and the first Luer connector (14) are connected such that when the inner tube (9) is inserted into and assembled with the outer tube (10), the relative positions of the inner tube (9) and the outer tube (10) are fixed.

10. The inserting apparatus of any of claims 6 to 9, wherein surfaces of the inner tube (9) and the outer tube (10) are provided with a coating that improves patient comfort and safety.
